(19) Europäisches Patentamt
European Patent Office
Office européen des brevets

(11) EP 2 442 784 B1

(12) # EUROPEAN PATENT SPECIFICATION

(45) Date of publication and mention of the grant of the patent:
**01.05.2013 Bulletin 2013/18**

(21) Application number: **10737442.3**

(22) Date of filing: **22.07.2010**

(51) Int Cl.:
***A61K 31/198*** (2006.01) ***A61K 31/445*** (2006.01)
***A61Q 19/02*** (2006.01) ***A61K 8/44*** (2006.01)
***A61K 8/67*** (2006.01) ***A61K 36/03*** (2006.01)
***A61K 8/97*** (2006.01)

(86) International application number:
**PCT/US2010/042861**

(87) International publication number:
**WO 2012/011908 (26.01.2012 Gazette 2012/04)**

(54) **COMPOSITIONS AND METHODS FOR INHIBITING PAR2 ACTIVATION OF KERATINOCYTES**

ZUSAMMENSETZUNGEN UND VERFAHREN ZUR HEMMUNG DER PAR2-AKTIVIERUNG VON KERATINOZYTEN

COMPOSITIONS ET PROCÉDÉS PERMETTANT D'INHIBER L'ACTIVATION DE PAR2 DANS LES KÉRATINOCYTES

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(43) Date of publication of application:
**25.04.2012 Bulletin 2012/17**

(73) Proprietor: **THE PROCTER & GAMBLE COMPANY**
**Cincinnati, OH 45202 (US)**

(72) Inventors:
- **SWANSON, Cheri Lynn**
  **West Chester**
  **Ohio 45069 (US)**
- **HAKOZAKI, Tomohiro**
  **Cincinnati**
  **Ohio 45249 (US)**
- **LAUGHLIN, Leo TImothy, II**
  **Mason**
  **Ohio 45040 (US)**

(74) Representative: **Engisch, Gautier**
**NV Procter & Gamble Services Company SA**
**IP Patent Department**
**Temselaan 100**
**1853 Strombeek-Bever (BE)**

(56) References cited:
**US-A1- 2005 019 356 US-A1- 2008 119 527**

- **BISSETT DONALD L ET AL: "Reduction in the appearance of facial hyperpigmentation by topical N-undecyl-10-enoyl-L-phenylalanine and its combination with niacinamide.", December 2009 (2009-12), JOURNAL OF COSMETIC DERMATOLOGY DEC 2009 LNKD- PUBMED: 19958429, VOL. 8, NR. 4, PAGE(S) 260 - 266, XP9144669, ISSN: 1473-2165 * abstract**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

FIELD OF THE INVENTION

The present invention relates to methods for boosting PAR2 inhibition by using a vitamin B3 compound, an N-acyl amino acid compound, and an extract of *Laminaria Saccharina.*

BACKGROUND OF THE INVENTION

Melanin is fundamental compound in skin pigmentation. Melanin is produced by a complex set of reactions within the melanocyte involving, at a basic level, the enzyme tyrosinase and L-tyrosine as a substrate. Tyrosinase catalyzes the conversion of L-tyrosine to DOPA (L-3,4-dihydroxyphenylalanine) and of DOPA to dopaquinone. Dopaquinone undergoes further conversion to form melanin. Melanin aggregates in organelles known as the melanosomes which are transferred to keratinocytes along slender filaments of the melanocyte known as dendrites. There are approximately 1500 gene products expressed in melanosomes with 600 of them being expressed at any given time and 100 of them believed to be unique to the melanosome. In addition, there are many regulatory elements involved in signaling, in the transport of melanosomes within the melanocyte, and in the transfer of melanosomes to the keratinocytes.

One mechanism in the melanin production cycle is the transfer of melanosomes from the melanocytes to the keratinocytes by way of phagocytosis. Research has found that the protease-activated receptor 2 (PAR-2) expressed on keratinocytes is involved in melanosome transfer and therefore may regulate pigmentation. *See* Seiberg, M. et al., The Protease-Activated Receptor 2 Regulates Pigmentation via Keratinocyte-Melanocyte Interactions, Experimental Cell Research 254, 25-32 (2000). Activation of PAR-2 with trypsin (or a trypsin-like protease) (or with the peptide agonist SLIGRL) induces pigmentation, which in some cases may manifest as a hyperpigmented spot or uneven skin tone. Therefore, compounds that inhibit trypsin (or a trypsin-like protease) activation of PAR-2 are believed to disrupt or reduce the phagocytosis of the melanocytes by the keratinocytes. Compounds that inhibit the PAR-2 activation will like regulate hyperpigmentation and melanin overproduction.

Conditions associated with overproduction of melanin are termed hyperpigmentation. For example, melanosis or melasma is a condition characterized by the development of sharply demarcated blotchy, brown spots usually in a symmetric distribution over the cheeks, forehead, and sometimes on the upper lip and neck. This condition frequently occurs during pregnancy (melasma gravidarum or "mask of pregnancy"), and at menopause. Also, this condition is frequently found among those taking oral contraceptives, and occasionally found among nonpregnant women who are not taking oral contraceptives, and sometimes among men. A pattern of facial hyperpigmentation, similar to that described above, may be associated with a chronic liver disease called chloasma. Another common condition associated with aging skin is the development of dark spots sometimes referred to as "age spots." Other forms of hyperpigmentation can be caused by UV irradiation or other inflammatory stimuli. Hyperpigmentation may also result from a genetic predisposition for the condition, or may come about during the course of wound healing.

Many active ingredients are marketed for improving the appearance of hyperpigmentation. For example, the use of N-acyl amino acid compounds such as N-acyl Phenylalanine and N-acyl Tyrosine and vitamin B3 have been shown to be beneficial in the regulation of melanin production *in vitro. See* Millikin, C.L. *et al.* (2008, February). *Undecyl-10-enoyl-phenylalanine (Sepiwhite®) provides additional benefits to the cosmetic ingredients N-acetyl glucosamine and niacinamide in the regulation of melanin production in vitro,* poster presented at 2008 American Academy of Dermatology, San Antonio, TX. While existing products are effective at regulating hyperpigmentation, there is an ongoing desire for more efficacious products.

Extracts of *Laminaria Saccharina,* a species of brown algae, are known in the art. One example is sold under the tradename Phlorogine by Biotech Marine, France. Phlorogine is known as anti-seborrhoeic agent that can regulate the activity of sebaceous glands, as described for example in United States Patent Application Publication No. 2008/0119527A1. Extraction methods for brown algae are also known. European Patent No. 1074262B1 describes an extraction method for the class *Phaeophyceae* and the species *Laminaria Ochroleuca.* These extracts are described as being used in cosmetic compositions as an osmoprotector, free-radical scavenger, or against the effects of skin aging effects. A cosmetic composition sold under the brand name SK-II Facial Clear Solution (Procter & Gamble, Cincinnati, OH) has a concentration of Phlorogine of about 1.25%. The SK-II Facial Clear Solution is marketed as a gel hydrator that moisturizes the skin without increasing oily shine.

SUMMARY OF THE INVENTION

A composition for inhibiting PAR2 activation of keratinocytes comprising a vitamin B3 compound, an N-acyl amino acid compound, and a *Laminaria Saccharina* extract. In certain embodiments, the composition for inhibiting PAR2 activation of keratinocytes comprises niacinamide, N-undecylenoyl-L-phenylalanine, and a *Laminaria Saccharina* extract

The invention further relates to methods for inhibiting PAR2 activation of keratinocytes by applying the disclosed composition. In response to the technical problems identified in the background, the present invention may take other forms. Further forms of the present invention will be appreciated from the detailed description that follows.

## BRIEF DESCRIPTION OF THE DRAWINGS

It is believed that the present invention will be better understood from the following description taken in conjunction with the accompanying drawings. The referenced drawings are not to be construed as limiting the scope of present invention.

Figure 1 is an exemplary applicator in the form of a dropper.
Figure 2 is an exemplary applicator in the form of a wand.
Figure 3 is an exemplary applicator in the form of a narrow-tip tube.
Figure 4 is a full color image of a participant.

## DETAILED DESCRIPTION OF THE INVENTION

All percentages and ratios used herein are by weight of the total composition and all measurements made are at 25°C, unless otherwise designated. All numeric ranges are inclusive of narrower ranges; delineated upper and lower range limits are interchangeable to create further ranges not explicitly delineated.

The compositions of the present invention can comprise, consist essentially of, or consist of, the essential components as well as optional ingredients described herein. As used herein, "consisting essentially of" means that the composition or component may include additional ingredients, but only if the additional ingredients do not materially alter the basic and novel characteristics of the claimed compositions or methods.

The term "apply" or "application", as used in reference to a composition, means to apply or spread the compositions of the present invention onto a human skin surface such as the epidermis.

The term "dermatologically acceptable," as used herein, means that the compositions or components described are suitable for use in contact with human skin tissue without undue toxicity, incompatibility, instability, allergic response, and the like.

The term "safe and effective amount" as used herein means an amount of a compound or composition sufficient to significantly induce a positive benefit.

The term "post-inflammatory hyperpigmentation" as used herein refers to an acute or temporary increase in pigmentation as a response to a transient inflammatory event, especially in dark skin subjects. Post-inflammatory hyperpigmentation typically subsides once the transient inflammatory event dissipates. Examples of transient inflammatory events include, but are not limited to, acne lesions, ingrown hairs, scratches, insect bites, surfactant damage, and short-term UV exposure.

The term "hyperpigmented spot" as used herein refers to a defined area of skin wherein the pigmentation is greater than that of an adjacent area of skin due to localized and chronic or systemic overproduction of melanin. Hyperpigmented spots typically are between about 2 mm and about 10 mm in diameter but smaller or larger spots are possible. Hyperpigmented spots can include one or more of age spots, sun spots, solar lentigos, hypo-melanotic lesions, freckles, and melasma spots.

The term "age spots" as used herein refers to a defined area of skin wherein the pigmentation is greater than that of adjacent skin due to localized and chronic overproduction of melanin caused by intrinsic or extrinsic aging factors.

The term "skin tone agent" as used herein refers to an agent that regulates melanin production signals, synthesis of melanin, systemic transfer of melanin between the melanocyte and the keratinocyte, and/or melanin degradation. Skin tone agents can improve the appearance of uneven skin tone by acting as a lightening or pigmentation reduction cosmetic agent.

The term "skin tone" as used herein refers to the overall appearance of melanin in the skin caused by the systemic, rather than transient, synthesis of melanin. Skin tone is typically characterized over a larger area of the skin. The area ideally may be than 100 $mm^2$, but larger areas are envisioned such as the entirety of the facial skin or any of the facial skin surfaces. Skin tone can be measured by image analysis. For example, overall lightness can be measured by L* coordinate in L*a*b* color space (International Commission on Illumination). Chromophore mapping such as melanin mapping and melanin concentration may be used as an indicator of overall skin tone. Mean melanin may be calculated from the chromophore map data. Additionally, skin tone evenness can be determined by melanin evenness which also may be calculated from the chromophore map data. Suitable chromophore mapping techniques are discussed in the example below.

The term "facial skin surfaces" as used herein refers to one or more of forehead, periorbital, cheek, perioral, chin, and nose skin surfaces.

I. Compositions

The compositions of the present invention are for the treatment, regulation, improvement, and/or prevention of hyperpigmentation. The compositions are for application to a mammalian skin surface and particularly to human skin. The compositions may be in a wide variety of product forms that include, but are not limited to, solutions, suspensions, lotions, creams, gels, toners, sticks, pencil, sprays, aerosols, ointments, cleansing liquid washes and solid bars, shampoos and hair conditioners, pastes, foams, powders, mousses, shaving creams, wipes, strips, patches, electrically-powered patches, wound dressing and adhesive bandages, hydrogels, film-forming products, facial and skin masks (with and without insoluble sheet), make-up such as foundations, eye liners, and eye shadows, and the like. The composition form may follow from the particular dermatologically acceptable carrier chosen, if present in the composition.

A. *Laminaria Saccharina* Extract

Compositions of the present invention include a safe and effective amount of *Laminaria Saccharina* extract, a brown algae extract. A preferred extract is Phlorogine and/or Phlorogine BG, which is available from Marine Biotech, France. Another suitable *Laminaria Saccharina* extract is available via product code HG 657 from Ennagram, France. The composition may contain Phlorogine and/or Phlorogine BG in an amount of at least about 0.001% 0.0006%, 0.2%, or 1%, by weight of the composition. The composition may contain Phlorogine and/or Phlorogine BG in an amount of less than about 50%, 20%, 10%, 5%, or 1%, by weight of the composition. Exemplary ranges for Phlorogine and/or Phlorogine BG use include from 0.008% to 50%, from about 0.04% to 20%, from 0.2% to 10%, from 1% to 5%, and from 1% to 3% by weight of the composition.

The composition may comprise a sufficient amount of *Laminaria Saccharina* extract to boost or improve the hyperpigmentation treatment effect of other actives within the compositions. In one embodiment, the composition comprises a sufficient amount of *Laminaria Saccharina* extract to boost or improve the efficacy of a vitamin $B_3$ compound and/or an N-acyl amino acid compound. In certain embodiments, the *Laminaria Saccharina* extract may boost or improve the efficacy of a niacinamide and/or N-undecylenoyl-L-phenylalanine. In another embodiment, the composition may comprise a sufficient amount of *Laminaria Saccharina* extract to boost or improve the PAR-2 inhibitory effect of other actives within the compositions. In certain embodiments, the *Laminaria Saccharina* extract may boost or improve the PAR-2 inhibitory effect of a niacinamide and/or N-undecylenoyl-L-phenylalanine. In another embodiment, the composition comprises a sufficient amount of *Laminaria Saccharina* extract to boost PAR2 inhibitory effect of the other components of the composition. For example, the composition may comprise a sufficient amount of *Laminaria Saccharina* extract to boost the PAR2 inhibitory effect of the other components of the composition of the vitamin B3 compound and the N-acyl amino acid compound.

The *Laminaria Saccharina* extract may include other compounds, such as, for example water, thickeners, humectants, solvents and solubilizers, etc. For example, Phlorogine and/or Phlorogine BG contain approximately about 1% to about 2.5% dry extract with the remaining material being inert carrier. The composition of the present invention therefore may contain a *Laminaria Saccharina* extract in an amount from about 0.00001% to about 1.25%, in one embodiment from about 0.00006% to about 0.5%, in another embodiment from about 0.002% to about 0.25%, by weight of the composition. In yet another embodiment the composition comprises from about 0.01% to about 0.125%, and in yet another embodiment from 0.01% to 0.075% *Laminaria Saccharina* extract by weight of the total composition. The *Laminaria Saccharina* extract can be prepared by processes known in the art, such as, for example, described in European Patent No. 1074262B 1.

B. N-acyl Amino Acid Compound

The compositions may comprise a safe and effective amount of one or more N-acyl amino acid compounds. The amino acid can be one of any of the amino acids known in the art. N-acyl amino acid compound includes N-acyl amino acids, their isomers, their salts, and derivatives thereof. The N-acyl amino acid compounds of the present invention correspond to Formula I:

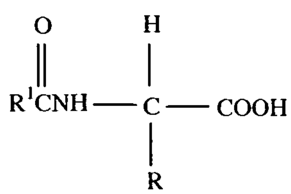

Formula I

wherein R can be a hydrogen, alkyl (substituted or unsubstituted, branched or straight chain), aryl, or a combination of alkyl and aromatic groups. A list of possible side chains of amino acids known in the art are described in Stryer, Biochemistry, 1981, published by W.H. Freeman and Company. R' can be $C_1$ to $C_{30}$, saturated or unsaturated, straight or branched, substituted or unsubstituted alkyls; substituted or unsubstituted aromatic groups; or mixtures thereof.

The N-acyl amino acid compound may be selected from the group consisting of N-acyl phenylalanine, N-acyl tyrosine, their isomers, their salts, and derivatives thereof. The amino acid can be the D or L isomer or a mixture thereof. N-acyl phenylalanine corresponds to the following Formula II:

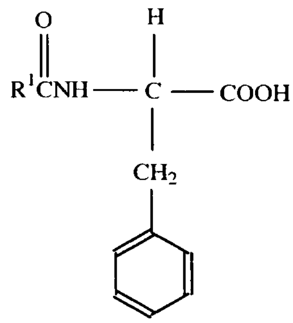

Formula II

wherein R' can be $C_1$ to $C_{30}$, saturated or unsaturated, straight or branched, substituted or unsubstituted alkyls; substituted or unsubstituted aromatic groups; or mixtures thereof.

N-acyl tyrosine corresponds to the following Formula III:

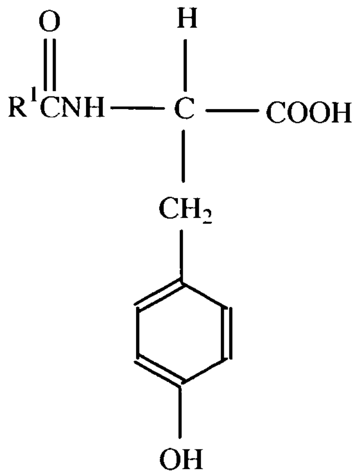

Formula III

wherein R' can be $C_1$ to $C_{30}$, saturated or unsaturated, straight or branched, substituted or unsubstituted alkyls; substituted or unsubstituted aromatic groups; or mixtures thereof.

Particularly useful as a topical skin tone evening (lightening or pigmentation reduction) cosmetic agent is N-undecylenoyl-L-phenylalanine. This agent belongs to the broad class of N-acyl phenylalanine derivatives, with its acyl group being a C11 mono-unsaturated fatty acid moiety and the amino acid being the L-isomer of phenylalanine. N-undecylenoyl-L-phenylalanine corresponds to the following Formula IV:

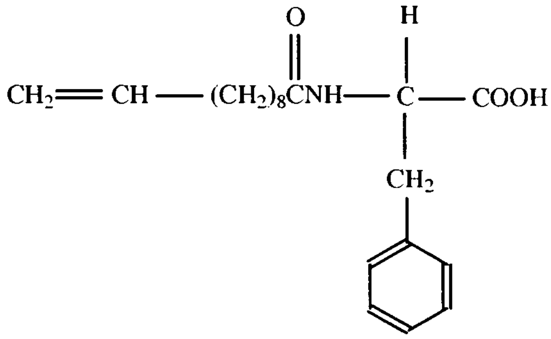

Formula IV

As used herein, N-undecylenoyl-L-phenylalanine is commercially available under the tradename Sepiwhite® from SEPPIC.

The composition of the present invention may comprise from about 0.0001 to about 25%, from about 0.001 to about 10%, from about 0.01 to about 5%, or from about 0.02 to about 2.5%, by weight of the composition, of the N-acyl amino acid.

C. Vitamin $B_3$

The compositions of the present invention may include a safe and effective amount of a vitamin B3 compound. Vitamin $B_3$ compounds are particularly useful for regulating skin condition as described in U.S. Patent No. 5,939,082. The composition contains from about 0.01% to about 50%, from about 0.1% to about 20%, from about 0.5% to about 10%, from about 1% to about 5%, or from about 2% to about 5%, by weight of the composition, of the vitamin B3 compound.

As used herein, "vitamin $B_3$ compound" means a compound having the Formula V:

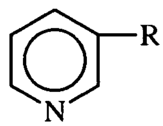

Formula V

wherein R is - CONH2 (i.e., niacinamide), - COOH (i.e., nicotinic acid) or - CH2OH (i.e., nicotinyl alcohol); derivatives thereof; and salts of any of the foregoing.

Exemplary derivatives of the foregoing vitamin B3 compounds include nicotinic acid esters, including non-vasodilating esters of nicotinic acid (e.g., tocopheryl nicotinate, myristyl nicotinate).

Examples of suitable vitamin B3 compounds are well known in the art and are commercially available from a number of sources (e.g., the Sigma Chemical Company, ICN Biomedicals, Inc., and Aldrich Chemical Company).

D. Dermatologically Acceptable Carrier

The compositions of the present invention may also comprise a dermatologically acceptable carrier (which may be referred to as "carrier") for the composition. The phrase "dermatologically acceptable carrier", as used herein, means that the carrier is suitable for topical application to the keratinous tissue, has good aesthetic properties, is compatible with the actives in the composition, and will not cause any unreasonable safety or toxicity concerns. In one embodiment, the carrier is present at a level of from about 50% to about 99%, about 60% to about 98%, about 70% to about 98%, or, alternatively, from about 80% to about 95%, by weight of the composition.

The carrier can be in a wide variety of forms. Non-limiting examples include simple solutions (e.g., aqueous, organic solvent, or oil based), emulsions, and solid forms (e.g., gels, sticks, flowable solids, or amorphous materials). In certain embodiments, the dermatologically acceptable carrier is in the form of an emulsion. Emulsion may be generally classified as having a continuous aqueous phase (*e.g.*, oil-in-water and water-in-oil-in-water) or a continuous oil phase (*e.g.*, water-in-oil and oil-in-water-in-oil). The oil phase of the present invention may comprise silicone oils, non-silicone oils such as hydrocarbon oils, esters, ethers, and the like, and mixtures thereof.

The aqueous phase typically comprises water. However, in other embodiments, the aqueous phase may comprise components other than water, including but not limited to water-soluble moisturizing agents, conditioning agents, anti-microbials, humectants and/or other water-soluble skin care actives. In one embodiment, the non-water component

of the composition comprises a humectant such as glycerin and/or other polyols. However, it should be recognized that the composition may be substantially (*i.e.*, less than 1% water) or fully anhydrous.

A suitable carrier is selected to yield a desired product form. Furthermore, the solubility or dispersibility of the components *(e.g., Laminaria Saccharina* extract, sunscreen active, additional components) may dictate the form and character of the carrier. In one embodiment, an oil-in-water or water-in-oil emulsion is preferred.

Emulsions may further comprise an emulsifier. The composition may comprise any suitable percentage of emulsifier to sufficiently emulsify the carrier. Suitable weight ranges include from about 0.1% to about 10% or about 0.2% to about 5% of an emulsifier, based on the weight of the composition. Emulsifiers may be nonionic, anionic or cationic. Suitable emulsifiers are disclosed in, for example, U.S. Patent 3,755,560, U.S. Patent 4,421,769, and McCutcheon's Detergents and Emulsifiers, North American Edition, pages 317-324 (1986). Suitable emulsions may have a wide range of viscosities, depending on the desired product form.

The carrier may further comprise a thickening agent as are well known in the art to provide compositions having a suitable viscosity and rheological character.

E. Optional Ingredients

In some embodiments, it may be desirable to include a skin tone agent in the composition in combination with the *Laminaria Saccharina* extract. The skin tone agents can be included to further improve overall skin tone. When present, the compositions of the present invention contain up to about 50%, 40%, 30%, 20%, 10%, 5%, or 3%, by weight of the composition, of the skin tone agent. When present, the compositions of the present invention contain at least about 0.001%, 0.01%, 0.1%, 0.2%, 0.5%, or 1%, by weight of the composition, of the skin tone agent. Suitable ranges include any combination of the lower and upper limits including suitable ranges from about 0.1% to about 50%; from about 0.2% to about 20%; or from about 1% to about 10%, by weight of the composition, of the skin tone agent. The amounts listed herein are only to be used as a guide, as the optimum amount of the skin tone agent will depend on the specific active selected since their potency does vary considerably.

Suitable skin tone agents include, but are not limited to, sugar amines, arbutin, deoxyarbutin, 1,3-dihydroxy-4-alkylbenzene such as hexylresorcinol, sucrose dilaurante, bakuchoil (4-[(1E, 3S)-3-ethenyl-3,7-dimethyl - 1,6 octadienyl] phenol or monterpene phenol), pyrenoine (available from Biotech Marine, France), panicum miliaceum seed extract, arlatone dioic acid, cinnamic acid, ferulic acid, achromaxyl, methyl nicotinamide, oil soluble licorice extract, folic acid, undecylenic acid (i.e., undecenoic acid), zinc undecylenate, thiamine (Vitamin B1) and its hydrochloride, L-tryptophan, helianthus annuus (sunflower) and vitis vinifera (grape) leaf extract, carnosine (i.e., dragosine), methyl gentisate, 1,2-hexandiol and 1,2-octandiol (i.e., combination sold as Symdiol 68 by Symrise AG, Germany), inositol, , koijic acid, hexamidine compounds, salicylic acid, and retinoids including retinol and retinyl propionate.

In certain embodiments, the additional skin tone agent is selected from sugar amines, hexamidine compounds, salicylic acid, 1,3-dihydroxy-4-alkylbenzene such as hexylresorcinol, and retinoids. As used herein, "sugar amine" includes isomers and tautomers of such and its salts (*e.g.*, HCl salt) and its derivatives. Examples of sugar amines include glucosamine, N-acetyl glucosamine, mannosamine, N-acetyl mannosamine, galactosamine, N-acetyl galactosamine, their isomers (*e.g.*, stereoisomers), and their salts (*e.g.,* HCl salt). As used herein, "hexaminide compound" means a compound having the Formula VI:

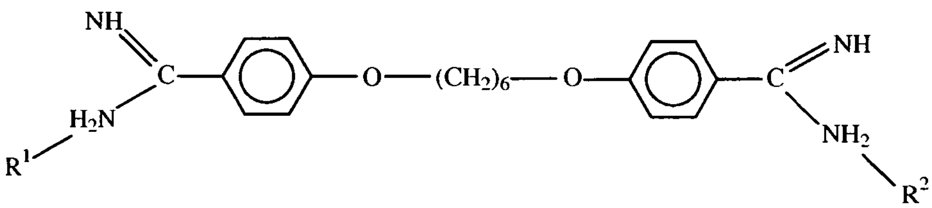

Formula VI

wherein $R^1$ and $R^2$ are optional or are organic acids (e.g., sulfonic acids, etc.). In one embodiment, hexamidine compound is hexamidine diisethionate.

Hyperpigmentation may result from skin inflammation. Transient inflammatory events triggering hyperpigmentation and, more specifically, post-inflammatory hyperpigmentation include, but are not limited to, acne lesions, ingrown hairs, scratches, insect bites, surfactant damage, allergens, and short-term UV exposure. Inflammation induced hyperpigmentation including post-inflammatory hyperpigmentation may be managed by incorporating into the compositions of the present invention an anti-inflammatory agent. When present, the compositions of the present invention contain up to about 20%, 10%, 5%, 3%, or 1% by weight of the composition, of the anti-inflammatory agent. When present, the

compositions of the present invention contain at least about 0.001%, 0.01%, 0.1%, 0.2%, 0.3%, 0.5%, or 1%, by weight of the composition, of the anti-inflammatory agent. Suitable ranges include any combination of the lower and upper limits. Suitable anti-inflammatory agents include, but are not limited to nonsteroidal anti-inflammatory agents (NSAIDS including but not limited to ibuprofen, naproxen, flufenamic acid, etofenamate, aspirin, mefenamic acid, meclofenamic acid, piroxicam and felbinac), glycyrrhizic acid (also known as glycyrrhizin, glycyrrhixinic acid, and glycyrrhetinic acid glycoside) and salts such as dipotassium glycyrrhizate, glycyrrhetenic acid, licorice extracts, bisabolol (*e.g.*, alpha bisabolol), manjistha (extracted from plants in the genus *Rubia,* particularly *Rubia cordifolia),* and guggal (extracted from plants in the genus *Commiphora,* particularly *Commiphora mukul),* kola extract, chamomile, red clover extract, and sea whip extract (extracts from plant in the order *Gorgonacea*), derivatives of any of the foregoing, and mixtures thereof.

The compositions of the subject invention may comprise one or more sunscreen actives (or sunscreen agents) and/or ultraviolet light absorbers. Herein, "sunscreen active" collectively includes, sunscreen actives, sunscreen agents, and/or ultraviolet light absorbers. Sunscreen actives include both sunscreen agents and physical sunblocks. Sunscreen actives may be organic or inorganic. Examples of suitable sunscreen actives are disclosed in Personal Care Product Council's International Cosmetic Ingredient Dictionary and Handbook, Thirteenth Edition, as "sunscreen agents." Particularly suitable sunscreen actives are 2-ethylhexyl-p-methoxycinnamate (commercially available as PARSOL™ MCX), 4,4'-t-butyl methoxydibenzoyl-methane (commercially available as PARSOL™ 1789), 2-hydroxy-4-methoxybenzophenone, octyldimethyl-p-aminobenzoic acid, digalloyltrioleate, 2,2-dihydroxy-4-methoxybenzophenone, ethyl-4-(bis(hydroxypropyl))aminobenzoate, 2-ethylhexyl-2-cyano-3,3-diphenylacrylate, 2-ethylhexyl-salicylate, glyceryl-p-aminobenzoate, 3,3,5-tri-methylcyclohexylsalicylate, menthyl anthranilate, p-dimethyl-aminobenzoic acid or aminobenzoate, 2-ethylhexyl-p-dimethyl-amino-benzoate, 2-phenylbenzimidazole-5-sulfonic acid, 2-(p-dimethylaminophenyl)-5-sulfonicbenzoxazoic acid, octocrylene, zinc oxide, benzylidene camphor and derivatives thereof, titanium dioxide, and mixtures thereof.

In one embodiment, the composition may comprise from about 1% to about 20%, and alternatively from about 2% to about 10% by weight of the composition, of the sunscreen active. Exact amounts will vary depending upon the chosen sunscreen active and the desired Sun Protection Factor (SPF), which is within the knowledge of one of skilled in the art.

The compositions of the present invention may contain a variety of other ingredients provided that they do not unacceptably alter the benefits of the invention. When present, compositions of the present invention may contain from about 0.0001% to about 50%; from about 0.001% to about 20%; or, alternately, from about 0.01% to about 10%, by weight of the composition, of the optional components. The amounts listed herein are only to be used as a guide, as the optimum amount of the optional components used in a composition will depend on the specific active selected since their potency does vary considerably. Hence, the amount of some optional components useful in the present invention may be outside the ranges listed herein.

The optional components, when incorporated into the composition, should be suitable for use in contact with human skin tissue without undue toxicity, incompatibility, instability, allergic response, and the like. The compositions of the present invention may include optional components such as anti-acne actives, desquamation actives, anti-cellulite agents, chelating agents, flavonoids, tanning active, non-vitamin antioxidants and radical scavengers, hair growth regulators, anti-wrinkle actives, anti-atrophy actives, minerals, phytosterols and/or plant hormones, N-acyl amino acid compounds, antimicrobial or antifungal actives, and other useful skin care actives, which are described in further detail in U.S. application publication No. US2006/0275237A1 and US2004/0175347A1.

The Personal Care Product Council's International Cosmetic Ingredient Dictionary and Handbook, Thirteenth Edition, describes a wide variety of non-limiting cosmetic and pharmaceutical ingredients commonly used in the skin care industry, which are suitable optional components for use in the compositions of the present invention. Examples of these ingredient classes include: abrasives, absorbents, aesthetic components such as fragrances, pigments, colorings/colorants, essential oils, anti-caking agents, antifoaming agents, antimicrobials, binders, biological additives, buffering agents, bulking agents, chelating agents, chemical additives, colorants, cosmetic astringents, cosmetic biocides, denaturants, drug astringents, emollients, external analgesics, film formers or materials, opacifying agents, pH adjusters, preservatives, propellants, reducing agents, sequestrants, skin cooling agents, skin protectants, thickeners viscosity modifiers, vitamins, and combinations thereof.

## II. Exemplary Compositions

The following are non-limiting examples of the compositions of the present invention. The examples are given solely for the purpose of illustration and are not to be construed as limitations of the present invention, as many variations thereof are possible without departing from the spirit and scope of the invention, which would be recognized by one of ordinary skill in the art. In the examples, all concentrations are listed as weight percent, unless otherwise specified and may exclude minor materials such as diluents, filler, and so forth. The listed formulations, therefore, comprise the listed components and any minor materials associated with such components. As is apparent to one of ordinary skill in the

art, the selection of these minor materials will vary depending on the physical and chemical characteristics of the particular ingredients selected to make the present invention as described herein.

All Examples may be used for the treatment, regulation, improvement, and/or prevention of hyperpigmentation. The present invention may further relate to a regimen involving the localized treatment for one or more hyperpigmented spots by a composition or to a regimen involving a more broad or general facial skin treatment by a composition.

| Component | Ex. A | Ex. B | Ex. C | Ex. D | Ex. E |
|---|---|---|---|---|---|
| Phlorogine or Phlorogine BG *1 | 2.000 | 1.000 | 1.000 | 1.000 | 1.000 |
| Niacinamide | 5.000 | 5.000 | 5.000 | 5.000 | 5.000 |
| Undecylenoyl-phenylalanine *2 (neutralized) | 1.000 | 1.000 | 0.500 | 1.000 | 1.000 |
| N-Acetylglucosamine | 0 | 0 | 2.000 | 0 | 0 |
| Hexamidine Diisethionate | 0 | | | 0.090 | 0.090 |
| Dipotassium Glycyrrhizate | 0 | 0.300 | 0.100 | 0.100 | 0.100 |
| Isohexadecane | 3.000 | 3.000 | 3.000 | 3.000 | 3.000 |
| Isopropyl isostearate | 1.330 | 1.330 | 1.330 | 1.330 | 1.330 |
| Cetearyl glucoside + cetearyl alcohol *3 | 0.200 | 0.200 | 0.200 | 0.200 | 0.200 |
| Behenyl alcohol | 0.400 | 0.400 | 0.400 | 0.400 | 0.400 |
| Cetyl alcohol | 0.320 | 0.320 | 0.320 | 0.320 | 0.320 |
| Stearyl alcohol | 0.480 | 0.480 | 0.480 | 0.480 | 0.480 |
| Tocopheryl acetate | 0.500 | 0.500 | 0.500 | 0.500 | 0.500 |
| PEG-100 stearate | 0.100 | 0.100 | 0.100 | 0.100 | 0.100 |
| Glvcerin | 7.000 | 7.000 | 7.000 | 7.000 | 7.000 |
| Polyacrylamide + C13-14 isoparaffin + laureth-7 *4 | 2.000 | 2.000 | 2.000 | 2.000 | 2.000 |
| Disodium EDTA | 0.100 | 0.100 | 0.100 | 0.100 | 0.100 |
| Benzvl alcohol | 0.400 | 0.400 | 0.400 | 0.400 | 0.400 |
| Dimethicone/ Dimethiconol *5 | 2.000 | 2.000 | 2.000 | 2.000 | 2.000 |
| Homosalate | 0 | 0 | 0 | 0 | 9.000 |
| Avobenzone | 0 | 0 | 0 | 0 | 3.000 |
| Octocrvlene | 0 | 0 | 0 | 0 | 2.600 |
| Oxybenzone | 0 | 0 | 0 | 0 | 1.000 |
| Octisalate | 0 | 0 | 0 | 0 | 4.500 |
| Water | QS | QS | QS | QS | QS |
| TOTAL | 100 | 100 | 100 | 100 | 100 |

*1 - Available from Biotech Marine, France.
*2 - Sepiwhite available from SEPPIC, France.
*3 - Emulgade PL 68/50 available from Cognis GmbH.
*4 - Sepigel 305, available from SEPPIC, France.
*5 - Dow Coming DC1503 available from Dow Coming, Inc., Midland, MI.

The compositions of the present invention are generally prepared by conventional methods such as are known in the art of making topical compositions. Such methods typically involve mixing of the ingredients in one or more steps to a relatively uniform state, with or without heating, cooling, application of vacuum, and the like. Typically, emulsions are prepared by first mixing the aqueous phase materials separately from the fatty phase materials and then combining the two phases as appropriate to yield the desired continuous phase. The compositions are preferably prepared such as to optimize stability (physical stability, chemical stability, photostability) and/or delivery of the active materials. This

optimization may include appropriate pH (e.g., less than 7), exclusion of materials that can complex with the active agent and thus negatively impact stability or delivery (e.g., exclusion of contaminating iron), use of approaches to prevent complex formation (e.g., appropriate dispersing agents or dual compartment packaging), use of appropriate photostability approaches (e.g., incorporation of sunscreen/sunblock, use of opaque packaging), etc.

III. Methods of Treatment

Various methods of treatment, application, regulation, or improvement may utilize the aforementioned compositions. The composition may be applied to a skin surface. The composition may be applied to a skin surface in need of PAR2 inhibition. For examples, the composition may be applied to a skin surface comprising a hyperpigmented spot or uneven tone. The composition may be applied to a skin surface in need of melanin regulation and/or reduction. Skin surfaces of the most concern tend to (but are not limited to) be those not typically covered by clothing such as facial skin surfaces, hand and arm skin surfaces, foot and leg skin surfaces, and neck and chest skin surfaces (*e.g.*, décolletage). In particular, identification of the hyperpigmented spot may be on a facial skin surface including the forehead, perioral, chin, periorbital, nose, and/or cheek skin surfaces.

The method may include the step of identifying a skin surface for treatment by the composition. The hyperpigmented spot may be identified by the user or a third party such as a dermatologist, cosmetician, or other caregiver. Identification may be done by visual inspection of the skin for hyperpigmented spots in need of treatment based on size and/or color. Identification of melanin may be done by commercially available imaging devices such SIAscope® V (available from Astron Clinica, Ltd., UK) or the VISIA® Complexion Analysis system (available from Canfield Scientific, Inc., Fairfield, NJ). Both devices are capable of collecting images of the skin and identifying chromophores such as melanin. These images can then be used to identify hyperpigmented spots or uneven tone.

Many dosing regimens exist for the application of the composition to a skin surface. The composition may be applied at least once a day, twice a day, or on a more frequent daily basis, during a treatment period. When applied twice daily, the first and second applications are separated by at least 1 to about 12 hours. Typically, the composition may be applied in the morning and/or in the evening before bed.

The composition may be applied and left on the skin surface for a sufficient contact time and/or repeatedly applied a sufficient number of times to achieve the desired PAR2 inhibition. In certain embodiments, the contact time is greater than about 1 hour, 2 hours, 6 hours, 8 hours, 12 hours, or 24 hours. The contact time is time from application of the composition until the composition is removed. In certain embodiments, the composition may be removed by rinsing or washing the substrate.

The treatment period is ideally of sufficient time to yield PAR2 inhibition. The treatment period may be of sufficient time provide an improvement in the appearance of a hyperpigmented spot, a reduction in melanin, or evening of skin tone. The improvement may be a detectable reduction in size of the hyperpigmented spot, lightening of the hyperpigmented spot (*e.g.*, lighter in color), or decrease in melanin of the hyperpigmented spot. The treatment period may involve a single application or multiple applications. The composition may be applied at least daily. In other embodiments, the composition is applied at least twice daily. Multiple applications may occur over the course of at least about 1 week. Alternately, the treatment period may last more than about 4 weeks or more than about 8 weeks. In certain embodiments, the treatment period will extend over multiple months (*i.e.*, 3-12 months) or multiple years.

The step of applying the composition to the skin surface may be done by localized application. In reference to application of the composition, the term "localized", "local", or "locally" mean that the composition is delivered the targeted area (such as the hyperpigmented spot) while minimizing delivery to skin surface not requiring treatment. It is recognized that localized application does allow for a reasonable amount of the composition to be applied to areas adjacent the targeted area such as a hyperpigmented spot (*i.e.*, the composition is unlikely to be applied or to remain within the boundary of the hyperpigmented spot without some spreading). The form of the composition or the dermatologically acceptable carrier should be selected to facilitate localized application. While certain embodiments of the present invention contemplate applying a composition locally to a hyperpigmented spot, it will be appreciated that compositions of the present invention can be applied more generally or broadly to one or more facial skin surfaces to reduce the appearance of hyperpigmented spots within those facial skin regions.

In some embodiments, the composition may be delivered by a variety of applicators appropriate for localized and general application. Several applicators are shown, by way of example, in Figures 1-3. In Figure 1, a suitable applicator 10 may be a dropper 12 which is shown with a bottle 14 that may contain the composition. Figure 2 shows an applicator 20 as a wand 22 with a housing 24 that may contain the composition. The wand 22 may comprise a handle 26, a stem 27, and an applicator head 28. The applicator head 28 may comprise fibers, foam, cotton, or any other suitable material that may releasably hold the composition. Figure 3 shows an applicator 30 as a narrow-tip tube 32 with a body 34 and narrow dispensing tip 36. The composition may be stored within the body 34 and dispensed through the pointed tip 36. Other applicators that can apply first composition locally to the hyperpigmented spot may also be used such as a cotton swab. Other suitable applicators include SH-0127 pen applicator available from Shya Hsin Plastic Works, Inc.,

Taiwan and either the Xpress Tip or liquid filled swab available from SwabPlus, Inc., China. The applicator may be configured to easily apply the composition to hyperpigmented spots having an approximate diameter between about 2 mm and about 10 mm and allowing for dosed amount of the composition of between about 1 to about 50 $uL/cm^2$ or between about 1 to about $5uL/cm^2$. In another embodiment, the composition is applied to the one or more hyperpigmented spots and more generally to one or more facial skin surfaces contemporaneously (*i.e.*, over a period of less than 30 minutes or, more typically, less than 5 minutes).

While some methods described herein contemplate applying the compositions of the present invention with an applicator, it will be appreciated that applicators are not required and the compositions of the present invention can also be applied directly by using one's finger or in other conventional manners.

Suitable methods may comprise any one or more of the abovementioned steps. One suitable method for inhibiting PAR2 activation of keratinocytes comprises the step of applying a composition comprising a vitamin B3 compound, an N-acyl amino acid compound, and a *Laminaria Saccharina* extract to a skin surface for a period of time sufficient to inhibit PAR2 activation of the keratinocytes. Another suitable method of inhibiting PAR2 activation of keratinocytes method comprises the step of applying a composition comprising a vitamin B3 compound, an N-acyl amino acid compound, and a *Laminaria Saccharina* extract to a skin surface, wherein the composition is applied at least daily for a period of time sufficient to inhibit PAR2 activation of the keratinocytes.

IV. Test Methods

The following methods are provided to illustrate certain features and advantages of various embodiments of the invention and should not be construed as limiting the scope thereof.

PAR-2 Inhibition Assay - The assay principle relies on the recruitment of beta arrestin to the activated PAR-2 receptor resulting in the formation of an active beta galactosidase enzyme which can cleave a substrate causing a luminescent signal that can be quantitated. PAR-2 can be activated by either adding trypsin and also by adding SLIGRL peptide.

Cells expressing PAR-2 receptor coupled to the alpha fragment of beta galactosidase and expressing beta arrestin coupled to the beta fragment of beta galactosidase are obtained from DiscoveRx Corp., Freemont, CA. Cells are propagated in a culture medium of Dulbeccos Modified Eagle Medium supplemented with 10% heat-inactivated fetal bovine serum, 500 unit/mL penicillin, 500 ug/mL streptomycin, 800 ug/mL gentimicin, and 300 ug/mL hygromycin in T75 or T150 culture flasks (all media components available from Invitrogen Corp., Carlsbad, CA) in a $CO_2$ incubator at 37°C. When cells reach approximately 80% confluency, the cells are detached from the flask using Cell Dissociation Buffer (available from Invitrogen Corp., Carlsbad, CA as item number 13151-014). The cells are counted using a hemocytometer and plated into 384 well plates at 10000 cells/well in 20 uL/well of Opti-MEM® media (available from Invitrogen Corp., Carlsbad, CA). The cells are cultured for 24 -48 hours.

To assay potential inhibitors of PAR2 activation by trypsin, each well is treated with 1 uL of the test material in water. Each test material is run in triplicate. The cells are incubated for 30 minutes in the $CO_2$ incubator, after which 1 uL of trypsin is added (approximate 0.1 ug/uL). The cells are incubated for an hour in the $CO_2$ incubator after which the cell lysis substrate solution (11 uL/well) is added. The lysis substrate solution is available in the PathHunter™ Detection Kit (catalogue #93-0001) from DiscoveRx Corp., Freemont, CA, in which the components are mixed in the following ratio:

Assay Buffer: Substrate 1: Substrate 2 = 19:5:1

The plates are briefly centrifuged (approximately 5 minutes) at 800 X g to remove bubbles and are then incubated at room temperature for 1 hr. Luminescence is read for each well on an Envision™ 2101 Multilabel Reader (available form PerkinElmer, Inc., Boston, MA). The range is determined by wells with solvent control + trypsin (high control) and by wells with solvent control and no trypsin (low control). The percent PAR2 inhibition of each treatment is calculated by the following formula of the luminescence signals:

$$\left(1-\frac{[\text{treatment luminescence - low control luminescence}]}{[\text{high control luminescence - low control luminescence}]}\right)\times 100$$

Statistics are done in Excel by Microsoft, Redmond, WA, using the T-test function, single tail with uneven distribution functions.

Using the assay outlined above, various concentrations and combinations of *Laminaria Saccharina* extract (e.g., Phlorogine), N-acyl Amino Acid (e.g., Sepiwhite), and vitamin B3 (e.g., niacinamide) were tested. Stock compositions of 1% Phlorogine (*i.e.*, composition comprises approximately 0.025% - 0.01% *Laminaria Saccharina* extract), 1%

Sepiwhite, 5% niacinamide, and combinations thereof are prepared and diluted prior to use. Test runs are performed at dilution factors of 0.045, 0.023, and 0.006. For clarity purposes, the 1% Sepiwhite composition diluted at a factor of 0.045 yields a test compositing comprising 0.045% Sepiwhite.

The values reported in the table below are percent inhibition of PAR2 activation. All values reported in the table below are believed to be statistically significant with an approximate $p \leq 0.05$. Figure 4 is a plot of the data in table where the Y-axis is the percent PAR2 inhibition. For each compound of combination of compounds, the three bar plots represent the dilution factors of 0.045, 0.023, and 0.006. A value of 100% represents inhibition of PAR2 activation equivalent to the control where no trypsin initiator is present.

| Dilution Factor | 1% Phlorogine | 1% Sepiwhite | 5% Niacinamide | 1% Phlorogine + 5% Niacinamide | 1% Phlorogine + 1% Sepiwhite | 1% Sepiwhite +5% Niacinamide | 1% Phlorogine + 1% Sepiwhite + 5% Niacinamide |
|---|---|---|---|---|---|---|---|
| 0.045 | 0 | 53 | 20 | 28 | 40 | 91 | 100 |
| 0.023 | 6 | 35 | 14 | 23 | 24 | 62 | 71 |
| 0.006 | -1 | 14 | 5 | 11 | 10 | 14 | 22 |

The test compositions comprising Phlorogine alone show little to no inhibitory effect. Sepiwhite and niacinamide individually demonstrate an inhibitory effect which is pronounced at higher dilutions. Sepiwhite and niacinamide together demonstrate a prominent inhibitory effect especially at higher dilutions. The combination of Sepiwhite, niacinamide, and Phlorogine demonstrate an even greater inhibitory effect than the combination of Sepiwhite and niacinamide. This results is unexpected given that Phlorogine alone provides little to no inhibitory effect. Without being bound to theory, Phlorogine is believed to boost the PAR2 inhibitory effect of niacinamide/Sepiwhite. By inhibiting PAR2 activation, the transfer of melanosomes from the melanocytes to the keratinocytes by way of phagocytosis is likewise inhibited. It is believed that increased inhibition of PAR2 activation will manifest itself by reducing or limiting less melanin presence in the keratinocytes.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

Every document cited herein, including any cross referenced or related patent or application, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

**1.** A cosmetic method of treating, regulating, improving and/or prevention of hyperpigmentation by inhibiting PAR2 activation of keratinocytes method comprising the step of applying a composition comprising a vitamin B3 compound, an N-acyl amino acid compound, and a *Laminaria Saccharina* extract to a skin surface for a period of time sufficient to inhibit PAR2 activation of the keratinocytes.

**2.** The method of claim 1 wherein the skin surface is a facial skin surface.

**3.** The method according to any one of the preceding claims, wherein the composition is applied to a hyperpigmented spot on the skin surface.

4. The method according to any one of the preceding claims, wherein the composition is applied to the skin surface at least once or twice a day for at least about four or eight weeks.

5. The method according to any one of the preceding claims, wherein the composition further comprises a dermatologically acceptable carrier.

6. The method according to any one of the preceding claims, wherein the composition further comprises a tone agent, a sunscreen active, an anti-inflammatory active, or combinations thereof.

7. A method according to Claim 1 wherein the composition is applied at least daily with a contact time of at least one hour.

8. A composition for inhibiting PAR2 activation of keratinocytes comprising:

a. from about 0.5% to about 10% niacinamide,
b. from about 0.001 to about 10% N-undecylenoyl-L-phenylalanine, and
c. a sufficient amount of *Laminaria Saccharina* extract to boost PAR2 inhibition of the niacinamide and N-undecylenoyl-L-phenylalanine.

## Patentansprüche

1. Kosmetisches Verfahren zum Behandeln, Regulieren, Verbessern und/oder Verhindern einer Hyperpigmentierung durch Hemmung einer PAR2-Aktivierung der Keratinozyten, wobei das Verfahren den Schritt des Auftragens einer Zusammensetzung, die eine Vitamin B3-Verbindung, eine N-Acylaminosäure-Verbindung und einen *Laminaria-Saccharina*-Extrakt umfasst, auf eine Hautoberfläche über einen ausreichend langen Zeitraum umfasst, um die PAR2-Aktivierung der Keratinozyten zu hemmen.

2. Verfahren nach Anspruch 1, wobei die Hautoberfläche eine Gesichtshautoberfläche ist.

3. Verfahren nach einem der vorstehenden Ansprüche, wobei die Zusammensetzung auf eine hyperpigmentierte Stelle auf der Hautoberfläche aufgetragen wird.

4. Verfahren nach einem der vorstehenden Ansprüche, wobei die Zusammensetzung über einen Zeitraum von mindestens etwa vier bis acht Wochen mindestens ein Mal oder zwei Mal pro Tag auf die Hautoberfläche aufgetragen wird.

5. Verfahren nach einem der vorstehenden Ansprüche, wobei die Zusammensetzung ferner einen dermatologisch verträglichen Träger umfasst.

6. Verfahren nach einem der vorstehenden Ansprüche, wobei die Zusammensetzung ferner ein Tonisierungsmittel, einen Sonnenschutzwirkstoff, einen Entzündungshemmer oder Kombinationen davon umfasst.

7. Verfahren nach Anspruch 1, wobei die Zusammensetzung mindestens täglich unter einer Kontaktzeit von mindestens einer Stunde aufgetragen wird.

8. Zusammensetzung zum Hemmen der PAR2-Aktivierung von Keratinozyten, die Folgendes umfasst:

a. etwa 0,5% bis etwa 10% Niacinamid,
b. etwa 0,001 bis etwa 10% N-Undecylenoyl-L-phenylalanin und
c. eine ausreichende Menge an *Laminaria-Saccharina*-Extrakt zum Verstärken der PAR2-Hemmung des Niacinamids und des N-Undecylenoyl-L-phenylalanins.

## Revendications

1. Procédé cosmétique de traitement, régulation, amélioration et/ou prévention d'une hyperpigmentation en inhibant l'activation par PAR2 de kératinocytes, le procédé comprenant l'étape consistant à appliquer une composition comprenant un composé de vitamine B3, un composé d'acide N-acyl aminé, et un extrait de *Laminaria Saccharina*

sur une surface de la peau pendant une période de temps suffisante pour inhiber l'activation par PAR2 des kératinocytes.

**2.** Procédé selon la revendication 1, dans lequel la surface de la peau est une surface de la peau du visage.

**3.** Procédé selon l'une quelconque des revendications précédentes, dans lequel la composition est appliquée à un point hyperpigmenté sur la surface de la peau.

**4.** Procédé selon l'une quelconque des revendications précédentes, dans lequel la composition est appliquée sur la surface de la peau au moins une fois ou deux fois par jour pendant au moins environ quatre ou huit semaines.

**5.** Procédé selon l'une quelconque des revendications précédentes, dans lequel la composition comprend en outre un véhicule acceptable du point de vue dermatologique.

**6.** Procédé selon l'une quelconque des revendications précédentes, dans lequel la composition comprend en outre un agent de tonus, un agent actif contre les rayons solaires, un agent actif anti-inflammatoire, ou leurs combinaisons.

**7.** Procédé selon la revendication 1, dans lequel la composition est appliquée au moins une fois par jour avec un temps de contact d'au moins une heure.

**8.** Composition pour inhiber l'activation par PAR2 de kératinocytes comprenant :

a. d'environ 0,5 % à environ 10 % de niacinamide,
b. d'environ 0,001 à environ 10 % de N-undécylénoyl-L-phénylalanine, et
c. une quantité suffisante d'extrait de *Laminaria Saccharina* pour inciter l'inhibition par PAR2 du niacinamide et de la N-undécylénoyl-L-phénylalanine.

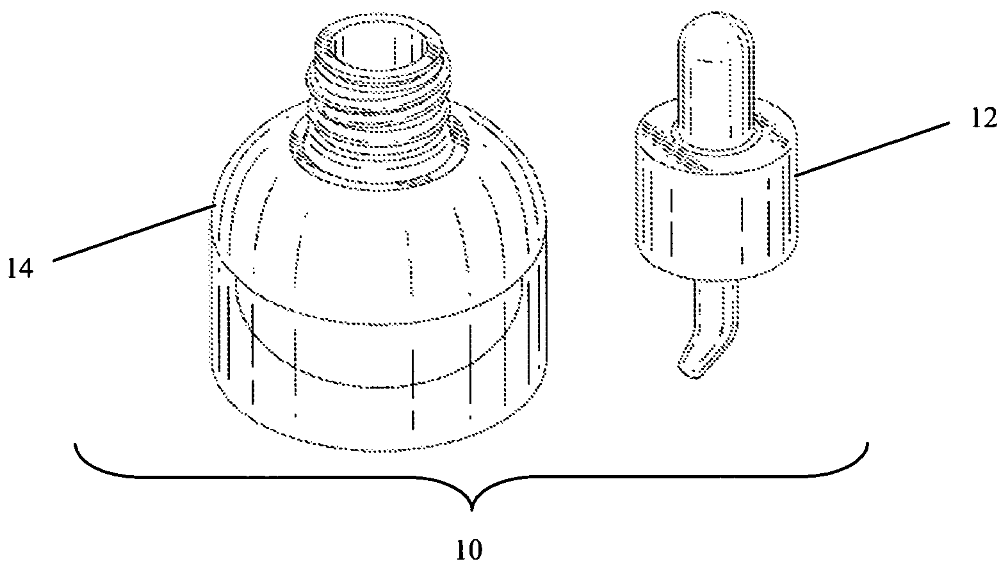


Fig. 1

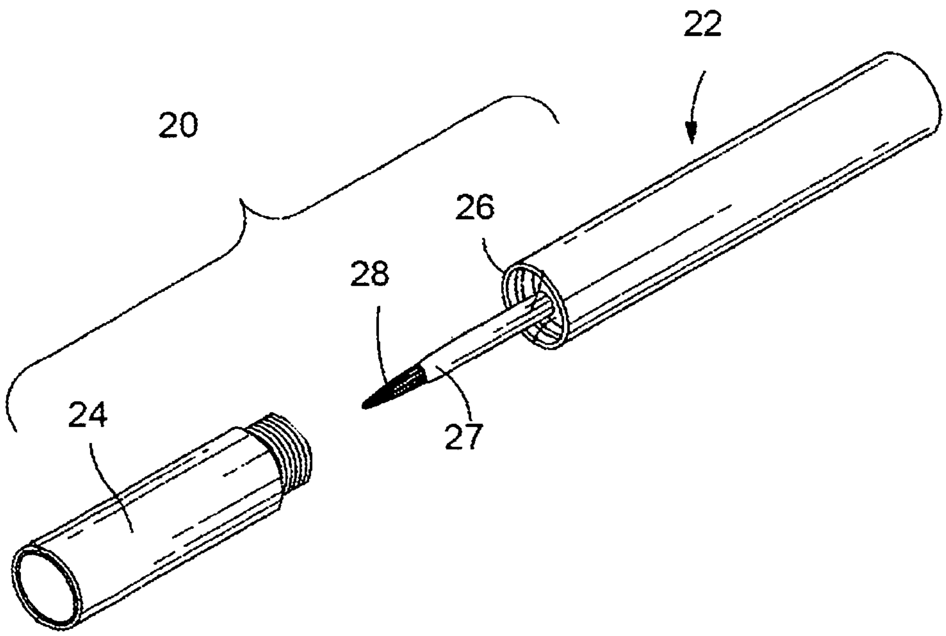


Fig. 2

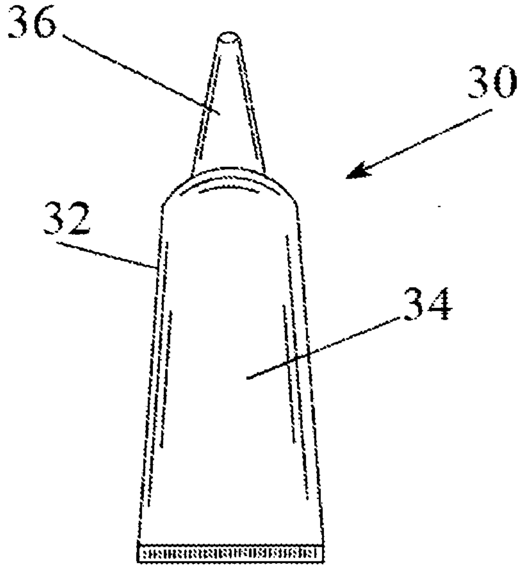


Fig. 3

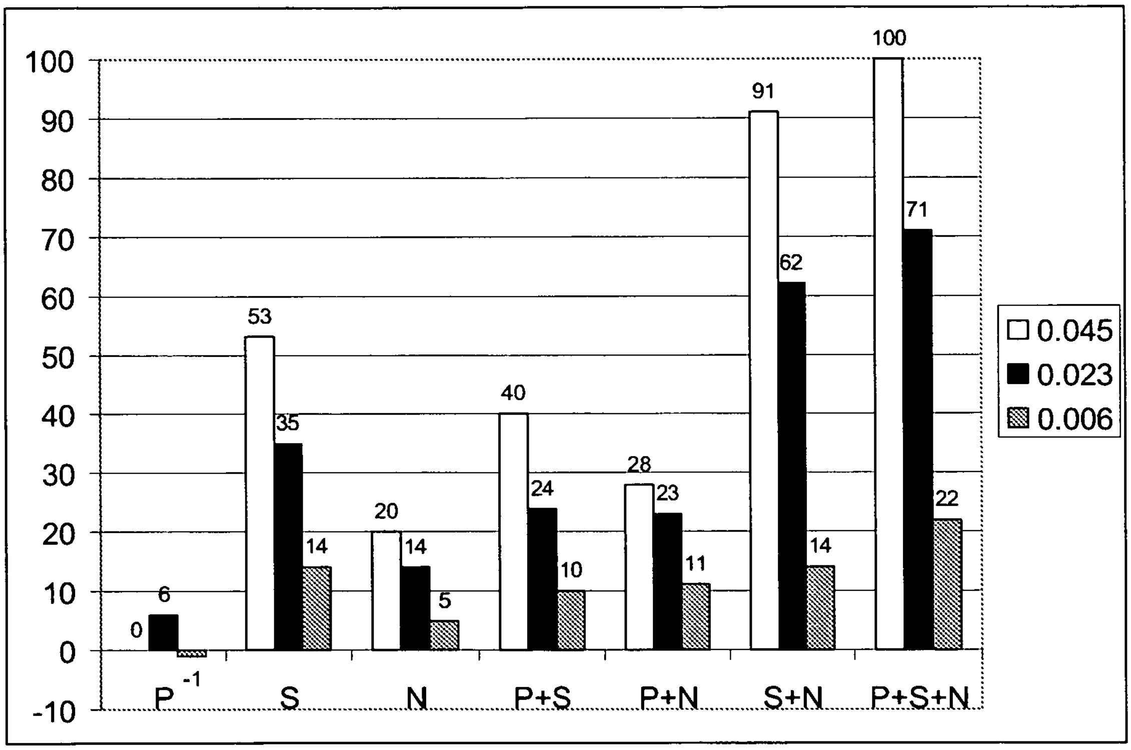
100
90
80
70
60
50
40
30
20
10
0
-10
0
6
-1
53
35
14
20
14
5
40
24
10
28
23
11
91
62
14
100
71
22
P
S
N
P+S
P+N
S+N
P+S+N
0.045
0.023
0.006

Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20080119527 A1
- EP 1074262 B1
- US 5939082 A
- US 3755560 A
- US 4421769 A
- US 20060275237 A1
- US 20040175347 A1


### Non-patent literature cited in the description


- **SEIBERG, M. et al.** The Protease-Activated Receptor 2 Regulates Pigmentation via Keratinocyte-Melanocyte Interactions. *Experimental Cell Research,* 2000, vol. 254, 25-32
- **STRYER.** Biochemistry. W.H. Freeman and Company, 1981
- McCutcheon's Detergents and Emulsifiers. 1986, 317-324
- sunscreen agents. Personal Care Product Council's International Cosmetic Ingredient Dictionary and Handbook
- The Personal Care Product Council's International Cosmetic Ingredient Dictionary and Handbook